# EUROPEAN PATENT APPLICATION

(11) **EP 4 223 879 A1**
(43) Date of publication of application: **09.08.2023**
(21) Application number: 21875747.4
(22) Date of filing: 29.09.2021
(51) Int. Cl.: C12N 15/56, C12N 1/15, C12N 1/19, C12N 1/21, C12N 5/10, C12N 9/28

(54) **ALPHA-AMYLASE VARIANT**

(30) Priority: 02.10.2020 JP 2020167797; 23.08.2021 JP 2021135746
(71) Applicant: Kao Corporation, Chuo-ku Tokyo 103-8210 (JP)
(72) Inventor: HIOKI, Takahiro, Wakayama-shi, Wakayama 640-8580 (JP); SHAKU, Mao, Wakayama-shi, Wakayama 640-8580 (JP); KAWAHARA, Akihito, Wakayama-shi, Wakayama 640-8580 (JP)
(74) Representative: Vossius & Partner Patentanwälte Rechtsanwälte mbB
(86) International application number: PCT/JP2021/036011
(87) International publication number: WO 2022/071451

(57) **Abstract**

Provided is an α-amylase which can function at low temperatures while maintaining or enhancing stability and/or cleaning performance. An α-amylase mutant, which is a mutant of a parent α-amylase, the α-amylase mutant comprising one or more modifications to amino acid residues at positions corresponding to positions G5, S38, T49, Q96, N126, T129, G140, F153, Q167, G179, W186, E187, N192, M199, Y200, L203, Y205, D206, R211, K215, H240, S241, Y242, G244, E257, F259, K278, H283, S284, A288, H295, Y296, N303, T320, S331, L348, Y360, W408, L429, V430, G433, A434, W439, N471, G476, and G477 in the amino acid sequence of SEQ ID NO: 2, the parent α-amylase or α-amylase mutant having at least 90% sequence identity to the amino acid sequence of SEQ ID NO: 4.

## Description

### Field of the Invention

The present invention relates to an **α**-amylase mutant.

### Background of the Invention

**α**-Amylases are used in a wide range of industries, such as starch, brewing, textiles, pharmaceuticals, and food, are known to have suitability for containing in cleaning agents, and are incorporated into dishwashing cleaning agents for automatic dishwashers and clothing cleaning agents as components removing starch stains.

Known **α**-amylases useful for cleaning agents are Bacillus sp. KSM-1378 (FERM BP-3048) strain-derived **α-**amylase AP1378 (Patent Literature 1), Termamyl and Duramyl (registered trademarks), which are *Bacillus licheniformis-derived* **α**-amylases, Bacillus sp. DSM12649 strain-derived **α**-amylase AA560 (Patent Literature 2), Bacillus sp. SP722 strain-derived **α**-amylase SP722 (SEQ ID NO: 4 of Patent Literature 3), Cytophaga-derived **α-**amylase CspAmy2 (Patent Literature 4), and the like. With regard to these **α**-amylases, mutants modified to improve their functions for specific applications, for example, mutants with enhanced stability in cleaning agents, have been reported (Patent Literature 5).

In recent years, from the viewpoint of environmental protection and cleaning cost reduction, it is important to reduce temperatures in dishwashing and laundry washing, particularly in laundry washing in laundries. In addition, shortening of the cleaning time is also desired. However, optimal temperatures of most enzymes, including amylases, are higher than temperatures generally set for low-temperature cleaning. For this reason, it is difficult to completely remove many starch stains.

Therefore, it is important to find **α**-amylases maintaining cleaning performance and amylolytic activity even at low temperatures, and having a high stain removal effect.

[Patent Literature 1] WO 94/26881
[Patent Literature 2] WO 00/60060
[Patent Literature 3] WO 06/002643
[Patent Literature 4] WO 2014/164777
[Patent Literature 5] WO 98/044126

### Summary of the Invention

The present invention relates to the following:
1) An **α**-amylase mutant, which is a mutant of a parent **α**-amylase, the **α**-amylase mutant comprising one or more modifications to amino acid residues at positions corresponding to positions G5, S38, T49, Q96, N126, T129, G140, F153, Q167, G179, W186, E187, N192, M199, Y200, L203, Y205, D206, R211, K215, H240, S241, Y242, G244, E257, F259, K278, H283, S284, A288, H295, Y296, N303, T320, S331, L348, Y360, W408, L429, V430, G433, A434, W439, N471, G476, and G477 in the amino acid sequence of SEQ ID NO: 2,
   the parent **α**-amylase or **α**-amylase mutant having at least 90% sequence identity to the amino acid sequence of SEQ ID NO: 4.
2) A polynucleotide encoding the mutant according to 1).
3) A vector or DNA fragment comprising the polynucleotide according to 2).
4) A transformed cell comprising the vector or DNA fragment according to 3).
5) A cleaning agent composition comprising the mutant according to 1).

### Brief Description of Drawings

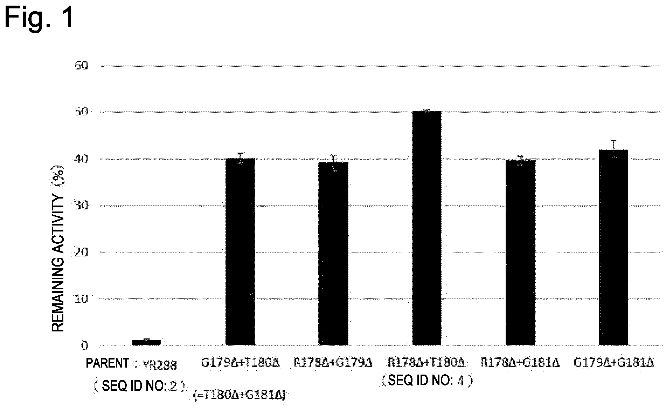

[Fig. 1] Fig. 1 shows the stability evaluation of mutants having a deletion of two amino acids.

### Detailed Description of the Invention

The present invention relates to the provision of an **α**-amylase which can function at low temperatures while maintaining or enhancing the stability and/or cleaning performance.

The present inventors succeeded in obtaining, by using an **α**-amylase which functions at low temperatures as a parent, an **α**-amylase mutant having enhanced cleaning performance and/or stability in comparison with the parent **α**-amylase.

The present invention can provide an **α**-amylase mutant having enhanced cleaning performance and/or stability in comparison with a parent **α**-amylase. Use of such an **α**-amylase mutant allows cleaning with excellent starch stain removal effect even at low temperatures.

In the present specification, "amylase" (EC3.2.1.1; **α**-D-(1**→**4)-glucan glucanohydrolase) refers to a group of enzymes that catalyze the hydrolysis of starch and other linear or branched 1,4-glycoside oligosaccharides or polysaccharides. The **α**-amylase activity can be determined by measuring the amount of reducing ends produced by the enzymatic degradation of starch. The determination method is not limited thereto; for example, the **α**-amylase activity can also be determined by measuring the release of dye by the enzymatic degradation of dye-crosslinked starch, such as Phadebas (Soininen, K., M. Ceska, and H. Adlercreutz. "Comparison between a new chromogenic α-amylase test (Phadebas) and the Wohlgemuth amyloclastic method in urine." Scandinavian journal of clinical and laboratory investigation 30.3 (1972): 291-297.).

In the present specification, the identity of amino acid sequences or nucleotide sequences is calculated by the Lipman-Pearson method (Science, 1985, 227: 1435-1441). Specifically, the identity is calculated by analysis using a homology analysis (Search homology) program of genetic information processing software GENETYX Ver. 12 at a unit size to compare (ktup) of 2.

The "amino acid residues" herein refer to 20 amino acid residues constituting protein, i.e., alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), and valine (Val or V).

Amino acid positions and mutants are herein denoted as shown below using the officially recognized IUPAC one-letter amino acid abbreviations.

An amino acid at a predetermined position is denoted as [amino acid, position]. For example, threonine at position 226 is denoted as "T226."

"Substitution" of an amino acid is denoted as [original amino acid, position, substituted amino acid]. For example, substitution of threonine at position 226 with alanine is expressed as "T226A."

"Deletion" of an amino acid is denoted as [original amino acid, position, Δ]. For example, deletion of serine at position 181 is expressed as "S181Δ."

"Insertion" of an amino acid is denoted as [original amino acid, position, original amino acid, inserted amino acid]. For example, insertion of lysine after glycine at position 195 is expressed as "G195GK." Insertion of multiple amino acids is denoted as [original amino acid, position, original amino acid, inserted amino acid 1, inserted amino acid 2; and the like]. For example, insertion of lysine and alanine after glycine at position 195 is expressed by "G195GKA."

Mutants including multiple modifications are denoted by using the addition symbol ("+"). For example, "R170Y+G195E" represents substitution of arginine at position 170 with tyrosine and substitution of glycine at position 195 with glutamic acid, respectively.

When it is possible to introduce different modifications at one position, the different modifications are divided by the diagonal ("/"). For example, "R170Y/E" represents substitution of arginine at position 170 with tyrosine or glutamic acid.

The "operable linkage" between a regulatory region, such as a promoter, and a gene herein means that the gene and the regulatory region are linked so that the gene can be expressed under the control of the regulatory region. Procedures for the "operable linkage" between the gene and the regulatory region are well known to a person skilled in the art.

The "upstream" and "downstream" relating to a gene herein refer to the upstream and downstream in the transcription direction of the gene. For example, "a gene located downstream of a promoter" means that the gene is present on the 3' side of the promoter in the DNA sense strand, and the upstream of a gene means a region on the 5' side of the gene in the DNA sense strand.

The term "original" used herein for a function, property, or trait of a cell is used to indicate that the function, property, or trait is inherent in the cell. In contrast, the term "foreign" is used to describe a function, property, or trait that is introduced from outside the cell, rather than being inherent in the cell. For example, a "foreign" gene or polynucleotide is a gene or polynucleotide introduced into the cell from outside. The foreign gene or polynucleotide may be derived from an organism of the same species as the cell into which the foreign gene or polynucleotide is introduced, or from an organism of a different species (i.e., heterologous gene or polynucleotide).

### <Mutant>

The mutant of the present invention is a mutant of a parent α-amylase, the mutant containing one or more modifications to amino acid residues at positions corresponding to G5, S38, T49, Q96, N126, T129, G140, F153, Q167, G179, W186, E187, N192, M199, Y200, L203, Y205, D206, R211, K215, H240, S241, Y242, G244, E257, F259, K278, H283, S284, A288, H295, Y296, N303, T320, S331, L348, Y360, W408, L429, V430, G433, A434, W439, N471, G476, and G477 of the amino acid sequence of SEQ ID NO: 2.

That is, the "mutant" means a polypeptide having **α-**amylase activity in which one or more amino acid residues at predetermined positions are modified in amino acids constituting the parent **α**-amylase. Such modifications of amino acid residues at predetermined positions are intended for enhancing the cleaning performance and/or stability in cleaning agents. Therefore, such a mutant has enhanced cleaning performance and/or stability in comparison with the parent **α**-amylase.

In the mutant of the present invention, modification sites (mutation positions) of amino acid residues are positions corresponding to positions G5, S38, T49, Q96, N126, T129, G140, F153, Q167, G179, W186, E187, N192, M199, Y200, L203, Y205, D206, R211, K215, H240, S241, Y242, G244, E257, F259, K278, H283, S284, A288, H295, Y296, N303, T320, S331, L348, Y360, W408, L429, V430, G433, A434, W439, N471, G476, and G477 in the amino acid sequence of SEQ ID NO: 2.

The amino acid sequence of SEQ ID NO: 2 constitutes α-amylase YR288, and the mutation positions in the mutant of the present invention are numbered based on the amino acid numbers of the amino acid sequence.

YR288 is a protein registered as WP_100346362.1 in the NCBI protein sequence database, and is a protein specified by the present applicant as an α-amylase having high amylolytic activity and cleaning performance at low temperatures (Japanese Patent Application No. 2020-121626).

The "corresponding position" on the amino acid sequence can be determined by aligning the target sequence and the reference sequence (the amino acid sequence of SEQ ID NO: 2 in the present invention) so as to give maximum homology. Alignment of the amino acid sequences can be performed using known algorithms, the procedure of which is known to a person skilled in the art. For example, alignment can be performed by using the Clustal W multiple alignment program (Thompson, J.D. et al., 1994, Nucleic Acids Res. 22: 4673-4680) with default settings. Alternatively, Clustal W2 and Clustal omega, which are revised versions of Clustal W, can also be used. Clustal W, Clustal W2, and Clustal omega are available on the website of, for example, the European Bioinformatics Institute (EBI [www.ebi.ac.uk/index.html]) or the Japan DNA Data Bank operated by National Institute of Genetics (DDBJ [www.ddbj.nig.ac.jp/searches-j.html]). A position in the target sequence that is aligned to arbitrary position in the reference sequence by the above alignment is regarded as the "position corresponding" to the arbitrary position.

A person skilled in the art can further refine the alignment of the amino acid sequences obtained above to optimize them. Such optimal alignment is preferably determined in consideration of the similarity of amino acid sequences, the frequency of inserted gaps, and the like. The similarity of amino acid sequences as mentioned herein refers to, when two amino acid sequences are aligned, the ratio (%) of the number of positions where the same or similar amino acid residues are present in both sequences to the number of full-length amino acid residues. Similar amino acid residues refer to, among the 20 amino acids constituting proteins, amino acid residues which have similar properties to each other in terms of polarity and charge, and which undergo so-called conservative substitution. A group consisting of such similar amino acid residues is well known to a person skilled in the art, and examples include, but are not limited to, arginine and lysine or glutamine; glutamic acid and aspartic acid or glutamine; serine and threonine or alanine; glutamine and asparagine or arginine; leucine and isoleucine; and the like.

In the present invention, the "parent **α**-amylase" means a standard **α**-amylase to be modified to bring about the mutant of the present invention. The parent may be a natural (wild-type) polypeptide or a mutant thereof.

In the present invention, the amino acid sequence of the parent **α**-amylase or **α**-amylase mutant has at least 90%, preferably at least 95%, more preferably at least 96%, even more preferably at least 97%, even more preferably at least 98%, and even more preferably at least 99% identity to the amino acid sequence of SEQ ID NO: 4.

The **α**-amylase consisting of the amino acid sequence of SEQ ID NO: 4 is an (R178Δ+T180Δ) **α**-amylase mutant having deletion of amino acid residues corresponding to R178 and T180 in an **α**-amylase consisting of the amino acid sequence of SEQ ID NO: 2 (YR288). As shown in the examples provided later, a mutant whose parent **α**-amylase is YR288 and has deletion of any two amino acid residues at positions corresponding to positions R178, G179, T180, and G181 in the amino acid sequence of SEQ ID NO: 2 has significantly enhanced stability in cleaning agents, in comparison with YR288. Therefore, any of **α**-amylase mutants consisting of the amino acid sequence of SEQ ID NO: 2 or an amino acid sequence having at least 90%, preferably at least 95%, more preferably at least 96%, even more preferably at least 97%, even more preferably at least 98%, and even more preferably at least 99% identity thereto, and having deletion of any two or more amino acid residues at positions corresponding to positions R178, G179, T180, and G181 in the amino acid sequence of SEQ ID NO: 2, including an **α**-amylase consisting of the amino acid sequence of SEQ ID NO: 4, can serve as parent α-amylases for the mutant of the present invention.

Examples of positions for the deletion of any two amino acid residues preferably include R178Δ+T180Δ, G179Δ+T180Δ, R178Δ+G179Δ, R178Δ+G181Δ, G179Δ+G181Δ, and the like; and more preferably R178Δ+T180Δ.

Other examples of **α**-amylases consisting of an amino acid sequence having at least 90% identity to the amino acid sequence of SEQ ID NO: 4 include DE0178 which is *Bacillus* flexus-derived **α**-amylase, RU2C which is Bacillus sp.-derived **α**-amylase, and the like (Japanese Patent Application No. 2020-121626).

The one or more modifications to amino acid residues at predetermined positions include substitution, insertion, and/or deletion of an amino acid residue. "Substitution" means that an amino acid at a position is substituted with a different amino acid, "deletion" means that an amino acid at a position is deleted, and "insertion" means that an amino acid is added so as to be adjacent to and directly continuous with an amino acid at a position.

In the present invention, the number of mutation sites can be one or more, but is preferably 2 or more, more preferably from 2 to 15, even more preferably from 3 to 15, and even more preferably from 5 to 10.

The mutant is preferably an **α**-amylase having at least 90% identity to the amino acid sequence of SEQ ID NO: 4, from the viewpoint of enhancing the cleaning performance or stability. The mutant may contain any number of conservative amino acid substitutions as long as it retains the properties as the above mutant.

Among the mutation sites represented by positions corresponding to positions G5, S38, T49, Q96, N126, T129, G140, F153, Q167, G179, W186, E187, N192, M199, Y200, L203, Y205, D206, R211, K215, H240, S241, Y242, G244, E257, F259, K278, H283, S284, A288, fH295, Y296, N303, T320, S331, L348, Y360, W408, L429, V430, G433, A434, W439, N471, G476, and G477 in the amino acid sequence of SEQ ID NO: 2, preferred from the viewpoint of enhancing stability are positions corresponding to positions N126, E187, N192, F205, R211, H240, S241, and Y242. Of these, examples of two or more suitable modifications include combinations of the following modifications a) to f):
a) a combination of one or more modifications to amino acid residues selected from the groups consisting of amino acid residues at positions corresponding to positions E187, F205, and H240, and modification to at least one or more amino acid residues selected from the groups consisting of amino acid residues corresponding to positions N126, N192, R211, S241, and Y242;
b) a combination of one or more modifications to amino acid residues selected from the groups consisting of amino acid residues at positions corresponding to positions E187 and H240, and one or more modifications to amino acid residues selected from the groups consisting of amino acid residues at positions corresponding to positions N126, N192, F205, R211, S241, and Y242;
c) a combination of one or more modifications to amino acid residues selected from the groups consisting of amino acid residues at positions corresponding to positions F205 and H240, and one or more modifications to amino acid residues selected from the groups consisting of amino acid residues at positions corresponding to positions N126, E187, N192, R211, S241, and Y242;
d) a combination of modification to an amino acid residue at position corresponding to position F205, and one or modifications to amino acid residues selected from the groups consisting of amino acid residues at position corresponding to positions N126, E187, N192, R211, H240, S241, and Y242;
e) a combination of modification to an amino acid residue at position corresponding to position H240, and one or more modifications to amino acid residues selected from the groups consisting of amino acid residues at position corresponding to positions N126, E187, N192, F205, R211, S241, and Y242: and
f) a combination of modification to an amino acid residue corresponding to position R211, and one or more modifications to amino acid residues selected from the groups consisting of amino acid residues at positions corresponding to positions N126, E187, N192, F205, H240, S241, and Y242.

Preferred embodiments of modifications to amino acid residues at positions corresponding to positions G5, S38, T49, Q96, N126, T129, G140, F153, Q167, G179, W186, E187, N192, M199, Y200, L203, Y205, D206, R211, K215, H240, S241, Y242, G244, E257, F259, K278, H283, S284, A288, H295, Y296, N303, T320, S331, L348, Y360, W408, L429, V430, G433, A434, W439, N471, G476, and G477 are shown below.

That is, G5 is preferably substituted with E, D, P, R, or K (G5E/D/P/R/K);
S38 is preferably substituted with N (S38N);
T49 is preferably substituted with Q (T49Q);
Q96 is preferably substituted with R or K (Q96R/K);
N126 is preferably substituted with Y (N126Y);
T129 is preferably substituted with I (T129I);
G140 is preferably substituted with Y, F, or W (G140Y/F/W);
F153 is preferably substituted with W (F153W);
Q167 is preferably substituted with E (Q167E);
G179 is preferably substituted with D or H (G179D/H);
W186 is preferably substituted with L (W186L);
E187 is preferably substituted with P (E187P);
N192 is preferably substituted with F (N192F);
M199 is preferably substituted with L, T, A, N, Q, S, V, or I (M199L/T/A/N/Q/S/V/I);
Y200 is preferably substituted with G (Y200G);
L203 is preferably substituted with Y, M, or F (L203Y/M/F);
Y205 is preferably substituted with F (Y205F);
D206 is preferably substituted with R, E, N, T, or G (D206R/E/N/T/G);
R211 is preferably substituted with L, V, or I (R211L/V/I);
K215 is preferably substituted with F (K215F);
H240 is preferably substituted with F (H240F);
S241 is preferably substituted with A, Q, D, L, Y, P, or H (S241A/Q/D/L/Y/P/H);
Y242 is preferably substituted with F (Y242F);
G244 is preferably substituted with K, W, L, or R (G244K/W/L/R);
E257 is preferably substituted with T (E257T);
F259 is preferably substituted with W (F259W);
K278 is preferably substituted with L, D, W, I, H, S, T, N, Q, V, A, Y, or F (K278L/D/W/I/H/S/T/N/Q/V/A/Y/F);
H283 is preferably substituted with Q (H283Q);
S284 is preferably substituted with W (S284W);
A288 is preferably substituted with F (A288F);
H295 is preferably substituted with Y (H295Y);
Y296 is preferably substituted with A (Y296A);
N303 is preferably substituted with R, E, S, G, V, D, T, or A (N303R/E/S/G/V/D/T/A);
T320 is preferably substituted with D or E (T320D/E);
S331 is preferably substituted with T (S331T);
L348 is preferably substituted with I (L348I);
Y360 is preferably substituted with C, M, L, or V (Y360C/M/L/V);
position W408 is preferably substituted with P (W408P);
L429 is preferably substituted with V (L429V);
V430 is preferably substituted with M (V430M);
G433 is preferably subjected to insertion of S after G (G433GS);
A434 is preferably substituted with V (A434V);
W439 is preferably substituted with R (W439R);
N471 is preferably substituted with T (N471T);
G476 is preferably substituted with A, P, E, S, F, R, or K (G476A/P/E/S/F/R/K); and
G477 is preferably substituted with E (G477E).

Next, preferred combinations of mutations contributing to the enhancement of cleaning performance are shown in the following Tables 1-1 to 1-4. Therefore, mutants having at least such mutation combinations particularly contribute to the enhancement of cleaning performance.

**[Table 1-1]**

| |
|---|
| G5R+W186L+E257T |
| G5R+E257T+G433GS |
| G5R+Y360L+G433GS |
| G5R+Q96K+E257T |
| G5R+Y360L+W408P |
| G5R+F259W+S284W |
| G5R+W 186L+F259W |
| G5R+S284W+T320E |
| Q96K+F259W+G433GS |
| Q96K+W 186L+W439R |
| Q96K+W 186L+E257T |
| Q96K+W408P+N471T |
| Q96K+E257T+W408P |
| Q96K+W408P+G433GS |
| Q96K+F259W+N471T |
| Q96K+Y360L+A434V |
| Q96K+F259W+W408P |
| W186L+E257T+Y360L |
| W186L+W408P+N471T |
| W186L+E257T+W408P |
| W186L+E257T+N471T |
| E257T+A434V+N471T |
| E257T+W408P+A434V |
| F259W+S284W+W439R |
| F259W+S284W+Y360L |
| F259W+T320E+G433GS |
| S284W+T320E+Y360L |
| S284W+W408P+G433GS |
| S284W+T320E+A434V |
| T320E+Y360L+G433GS |

**[Table 1-2]**

| |
|---|
| T320E+W439R+N471T |
| T320E+G433GS+N471T |
| T320E+Y360L+W439R |
| Y360L+A434V+N471T |
| G5R+S284W+W439R |
| Q96K+E257T+T320E+W408P |
| Q96K+E257T+W408P+A434V |
| Q96K+E257T+W408P+N471T |
| Q96K+E257T+T320E+W408P+A434V |
| Q96K+E257T+T320E+W408P+N471T |
| Q96K+T320E+Y360L+W408P+A434V |
| Q96K+T320E+Y360L+A434V+N471T |
| E257T+T320E+W408P+A434V+N471T |
| G5R+Q96K+W186L+E257T+T320E+W439R |
| G5R+Q96K+W186L+E257T+S284W+G433GS |
| G5R+F259W+S284W+T320E+W439R+N471T |
| G5R+Q96K+W186L+Y360L+W408P+G433GS |
| G5R+Q96K+W186L+E257T+W408P+W439R |
| G5R+Q96K+F259W+Y360L+W408P+W439R |
| G5R+E257T+F259W+S284W+Y360L+N471T |
| G5R+Q96K+W186L+F259W+Y360L+G433GS |
| G5R+F259W+S284W+T320E+Y360L+A434V |
| Q96K+E257T+F259W+T320E+G433GS+N471T |
| Q96K+W186L+E257T+S284W+W439R+N471T |
| Q96K+S284W+T320E+W408P+W439R+N471T |
| Q96K+W186L+E257T+W408P+A434V+N471T |
| Q96K+W186L+F259W+Y360L+W439R+N471T |
| G5R+Q96K+Y360L+W408P+G433GS |
| G5R+Q96K+F259W+Y360L+G433GS |
| Q96K+F259W+Y360L+W439R+N471T |

**[Table 1-3]**

| |
|---|
| Q96K+E257T+W408P+A434V+N471T |
| E257T+Y360L |
| E257T+Y360L+W408P |
| E257T+Y360L+G476K |
| E257T+Y360L+W408P+G476K |
| G5R+S38N |
| Q96K+S38N |
| S38N+E257T |
| S38N+F259W |
| S38N+S284W |
| S38N+T320D |
| S38N+T320E |
| S38N+Y360L |
| S38N+W408P |
| S38N+N471T |
| S38N+G476A |
| S38N+G476K |
| S38N+G476E |
| S38N+N471T+G476K |
| S38N+N471T+G476K+G477E |
| E257T+Y360C |
| E257T+Y360M |
| E257T+Y360V |
| E257T+G476K+Y360C |
| E257T+G476K+Y360M |
| E257T+G476K+Y360V |
| G5R+W 186L |
| G5R+E257T |
| G5R+Y360L |
| S284W+T320E |
| Q96K+F259W |

**[Table 1-4]**

| |
|---|
| Q96K+W186L |
| Q96K+W408P |
| G5R+S284W |
| G5R+W439R |
| S38N+S284W |
| S38N+T320D |

Next, preferred combinations of mutations contributing to the enhancement of stability are shown in the following Tables 2-1 to 2-4. Therefore, mutants having at least such mutation combinations particularly contribute to the enhancement of stability. Mutations obtained by combining the mutation combinations of Tables 1-1 to 1-4 with the mutation combinations of Tables 2-1 to 2-4 are also preferred in terms of enhancing cleaning performance and stability.

**[Table 2-1]**

| |
|---|
| F205Y+H240F+Y242F |
| N192F+F205Y+H240F+Y242F |
| E187P+N192F |
| H240F+Y242F |
| H240F+Y242F+S331T |
| N126Y+T129I+L203Y+F205Y |
| N126Y+T1291+H240F+Y242F+G244W |
| N126Y+T1291+H283Q+A288F |
| H240F+Y242F+G244W |
| H240F+G244W |
| Y242F+G244W |
| L203Y+F205Y |
| N126Y+T129I |
| H283Q+A288F |
| S241Q+Y242F |
| S241F+G244W |
| S241Q+Y242F+G244W |
| H240F+S241Q+G244W |
| H240F+S241Q+Y242F |
| T129I+E187P+G244W+S331T |
| S241Q+H283Q+A288F+S331T |
| N126Y+T1291+G140W+E187P |
| N126Y+G179D+N192F+L203Y |
| N126Y+F205Y+Y242F+A288F |
| L203Y+R211I+Y242F+H283Q |
| G179D+E187P+R211I+H240F |
| G140W+L203Y+Y242F+G244W |
| H240F+S241Q+Y242F+G244W |
| F205Y+H240F+S241Q |
| F205Y+H240F+S241Q+Y242F |

**[Table 2-2]**

| |
|---|
| F205Y+S241Q |
| E187P+H240F+S241Q |
| E187P+H240F+S241Q+Y242F |
| E187P+S241Q+Y242F |
| E187P+H240F+Y242F |
| N126Y+E187P |
| T129I+E187P |
| G140W+E187P |
| E187P+L203Y |
| E187P+F205Y |
| E187P+S241Q |
| E187P+Y242F |
| E187P+G244W |
| E187P+H283Q |
| E187P+S331T |
| N126Y+T129I+E187P |
| E187P+N192F+F205Y+H240F+Y242F |
| M199L+F205Y+R211I+H240F+S241Q+Y242F |
| H240F+S241Q |
| E187P+R211I |
| E187P+N192F+M199L |
| E187P+F205Y+H240F+Y242F |
| E187P+N192F+Y242F |
| E187P+N192F+R211I |
| E187P+N192F+M199L+R211I |
| E187P+N192F+F205Y+Y242F |
| E187P+N192F+F205Y+H240F+Y242F |

**[Table 2-3]**

| |
|---|
| N192F+S241Q |
| N192F+H240F+S241Q |
| N192F+F205Y+H240F+S241Q |
| N192F+F205Y+H240F+S241Q+Y242F |
| R211I+H240F+S241Q |
| R211I+H240F+S241Q+Y242F |
| F205Y+R211I+H240F+S241Q |
| F205Y+R211I+H240F+S241Q+Y242F |
| G179H+M199L+F205Y+R211I+H240F+S241Q+Y242F |
| G179H+F205Y+R211I+H240F+S241Q+Y242F |
| G179H+F205Y+H240F+S241Q+Y242F |
| G179H+E187P+N192F |
| E187P+M199L |
| M199L+H240F+S241Q |
| F205Y+H240F+S241A |
| F205Y+H240F+S241D |
| H240F+S241A |
| H240F+S241D |
| N126Y+R211I |
| N126Y+H240F |
| E187P+H240F |
| N192F+F205Y |
| N192F+R211I |
| N192F+H240F |
| F205Y+R211I |
| F205Y+H240F |
| R211I+H240F |
| R211I+S241Q |
| R211I+Y242F |

**[Table 2-4]**

| |
|---|
| N192F+M199L+F205Y+R211I+H240F+S241Q+Y242F |
| G179H+N192F+M199L+F205Y+R211I+H240F+S241Q+Y242F |
| N192F+F205Y+R211I+H240F+S241Q+Y242F |
| G179H+N192F+F205Y+R211I+H240F+S241Q+Y242F |
| N192F+R211I+H240F+S241Q+Y242F |
| N192F+F205Y+R211I+H240F+S241Q |
| E187P+K278L |
| E187P+K278D |
| E187P+K278W |
| E187P+K278I |
| E187P+K278H |
| E187P+K278S |
| E187P+K278T |
| E187P+K278N |
| E187P+K278Q |
| E187P+K278V |
| E187P+K278A |
| E187P+K278Y |
| E187P+K278F |
| E187P+S241L+K278W |
| E187P+S241Y+K278I |
| E187P+S241P+K278W |
| E187P+S241L+K278I |
| E187P+S241P+K278I |
| E187P+S241Y+K278W |
| E187P+S241F+K278W |
| E187P+S241Y+K278Y |
| E187P+S241Y+K278F |
| E187P+S241Y+K278H |
| E187P+S241Y+K278L |
| E187P+S241H+K278W |

Next, preferred combinations of mutations contributing to the enhancement of cleaning performance and stability are shown in the following Table 3. Therefore, mutants having at least such mutation combinations particularly contribute to the enhancement of cleaning performance and stability.

**[Table 3]**

| |
|---|
| G5R+E187P+N192F+S284W+W439R |
| G5R+E187P+N192F+Y360L+G433GS |
| Q96K+E187P+N192F+F259W+G433GS |
| F205Y+H240F+S241Q+Y242F+E257T+Y360L |
| F205Y+H240F+S241Q+Y242F+E257T+Y360L+W408P |
| F205Y+H240F+S241Q+Y242F+E257T+Y360L+G476K |
| F205Y+R211I+H240F+S241Q+Y242F+E257T+Y360L |
| F205Y+H240F+S241Q+Y242F+E257T+Y360L+W408P+G476K |
| M199L+F205Y+H240F+S241Q+Y242F+E257T+Y360L+W408P+G476K |
| M199L+F205Y+H240F+Y242F+E257T+Y360L+S241Q+G476K |
| M199L+F205Y+R211I+H240F+S241Q+Y242F+E257T+Y360L+W408P+G476K |
| M199L+F205Y+H240F+S241Q+Y242F+E257T+S331T+Y360L+W408P+G476K |
| N126Y+M199L+F205Y+H240F+S241Q+Y242F+E257T+Y360L+W408P+G476K |
| E187P+M199L+F205Y+H240F+S241Q+Y242F+E257T+Y360L+G476K+W408P |
| G5R+Q96K+E187P+N192F+M199L+Y242F+F259W+S331T+Y360L+W439R |
| G5R+Q96K+E187P+N192F+M199L+R211I+Y242F+F259W+Y360L+W439R |
| G179H+N192F+M199L+F205Y+R211I+H240F+S241Q+Y242F+E257T+Y360L+W408P+G476K |
| S38N+G179H+N192F+M199L+F205Y+R211I+H240F+S241Q+Y242F+N471T+G476K+G477E |
| Q96K+E187P+N192F+R211I+F259W+G433GS |
| S38N+M199L+F205Y+R211I+H240F+S241Q+Y242F+N471T+G476K+G477E |
| Q96K+G179H+N192F+M199L+F205Y+R211I+H240F+S241Q+Y242F+F259W+G433GS |
| E187P+N192F+R211I+E257T+Y360L+W408P+G476K |
| Q96K+M199L+F205Y+R211I+H240F+S241Q+Y242F+F259W+G433GS |
| G5R+M199L+F205Y+R211I+H240F+S241Q+Y242F+S284W+W439R |
| G5R+G179H+N192F+M199L+F205Y+R211I+H240F+S241Q+Y242F+S284W+W439R |

### <Polynucleotide encoding the mutant of the present invention>

The mutant of the present invention can be produced by using various mutagenesis techniques known in this technical field. For example, the mutant of the present invention can be produced by mutating a polynucleotide encoding an amino acid residue to be modified in a parent **α**-amylase gene encoding its standard amino acid sequence (reference **α**-amylase gene) to a polynucleotide encoding the modified amino acid residue, and then allowing the mutated gene to express a mutant.

The polynucleotide encoding the mutant of the present invention can be in the form of single- or double-stranded DNA, RNA, or an artificial nucleic acid, or may be cDNA or chemically synthesized intron-free DNA.

In the present invention, as the means for mutating an amino acid residue of the parent **α**-amylase, various mutagenesis techniques known in this technical field can be used. For example, the polynucleotide encoding the mutant of the present invention can be obtained by mutating, in a polynucleotide encoding the amino acid residue of the parent **α**-amylase (hereinafter also referred to as the "parent gene"), a nucleotide sequence encoding an amino acid residue to be mutated to a nucleotide sequence encoding the mutated amino acid residue.

Introduction of the target mutation into the parent gene can be basically performed by various site-directed mutagenesis methods well-known to a person skilled in the art. The site-directed mutagenesis method can be performed by any method, such as an inverse PCR method or an annealing method. It is also possible to use commercially available site-directed mutagenesis kits (e.g., Stratagene's QuickChange II Site-Directed Mutagenesis Kit and QuickChange Multi Site-Directed Mutagenesis Kit, or the like).

Most commonly, site-directed mutagenesis of the parent gene can be performed by using a mutation primer containing the nucleotide mutation to be introduced. The mutation primer may be designed to be annealed to a region containing a nucleotide sequence encoding an amino acid residue to be mutated in the parent gene, and to contain a nucleotide sequence having a nucleotide sequence (codon) encoding the mutated amino acid residue in place of the nucleotide sequence (codon) encoding the amino acid residue to be mutated. The nucleotide sequences (codons) encoding the unmutated or mutated amino acid residues can be appropriately recognized and selected by a person skilled in the art based on ordinary textbooks and the like. Alternatively, site-directed mutagenesis can also be performed by using a method in which DNA fragments, obtained by amplifying the upstream and downstream sides of the mutation site separately using two complementary primers containing the nucleotide mutation to be introduced, are linked into one by SOE (splicing by overlap extension)-PCR (Gene, 1989, 77(1): pp. 61-68).

A template DNA containing the parent gene can be prepared by extracting genome DNA from a microorganism producing the α-amylase described above by a standard method, or extracting RNA and synthesizing cDNA with reverse transcription. Alternatively, a corresponding nucleotide sequence may be chemically synthesized based on the amino acid sequence of the parent **α**-amylase, and used as the template DNA. A DNA sequence containing a base sequence encoding an **α**-amylase consisting of the amino acid sequence of SEQ ID NO: 4 is shown in SEQ ID NO: 3, and a DNA sequence containing a base sequence encoding an **α**-amylase consisting of the amino acid sequence of SEQ ID NO: 2 (YR288) is shown in SEQ ID NO: 1.

A mutation primer can be produced by well-known oligonucleotide synthesis methods, such as the phosphoramidite method (Nucleic Acids R4esearch, 1989, 17: 7059-7071). Such primer synthesis can also be performed using, for example, a commercially available oligonucleotide synthesizer (e.g., ABI). Using a primer set containing the mutation primer, site-directed mutagenesis as described above can be carried out using the parent gene as the template DNA, thereby obtaining a polynucleotide encoding the mutant of the present invention having the target mutation.

The polynucleotide encoding the mutant of the present invention can contain single- or double-stranded DNA, cDNA, RNA, or other artificial nucleic acids. The DNA, cDNA, and RNA may be chemically synthesized. The polynucleotide may contain nucleotide sequences of untranslated regions (UTRs) in addition to open reading frames (ORFs). The codon of the polynucleotide may be optimized depending on the species of the transformant used for the production of the mutant polypeptide of the present invention. Information on codons used by various organisms is available from the Codon Usage Database ([www.kazusa.or.jp/codon/]).

### <Vector or DNA fragment>

The obtained polynucleotide encoding the mutant of the present invention can be incorporated into a vector. The type of vector to contain the polynucleotide is not particularly limited, and any vector, such as a plasmid, phage, phagemid, cosmid, virus, YAC vector, or shuttle vector, may be used. The vector is not limited, but is preferably a vector which can be amplified in bacteria, preferably Bacillus bacteria (e.g., *Bacillus subtilis* or mutant strains thereof), and more preferably an expression vector which can induce the expression of transgenes in Bacillus bacteria. Among these, shuttle vectors, which are vectors replicable in Bacillus bacteria and any other organisms, can be preferably used in the recombinant production of the mutant of the present invention. Examples of preferred vectors include, but are not limited to, pHA3040SP64, pHSP64R, or pASP64 (JP-B-3492935), pHY300PLK (an expression vector which can transform both *Escherichia coli* and *Bacillus subtilis;* Jpn J Genet, 1985, 60: 235-243), pAC3 (Nucleic Acids Res, 1988, 16: 8732), and other shuttle vectors; pUB110 (J Bacteriol, 1978, 134: 318-329), pTA10607 (Plasmid, 1987, 18: 8-15), and other plasmid vectors which can be used in the transformation of Bacillus bacteria; and the like. Other usable examples include *Escherichia coli*-derived plasmid vectors (e.g., pET22b(+), pBR322, pBR325, pUC57, pUC118, pUC119, pUC18, pUC19, and pBluescript, and the like).

The above vector may contain a DNA replication initiation region or a DNA region containing a replication origin. Alternatively, in the above vector, a regulatory sequence, such as a promoter region for initiating the transcription of the gene, a terminator region, or a secretion signal region for secreting the expressed protein outside the cell, may be operably linked to the upstream of the polynucleotide encoding the mutant of the present invention (i.e., mutant gene). The phrase that a gene and a regulatory sequence are "operably linked" means that the gene and the regulatory region are arranged so that the gene can be expressed under the control of the regulatory region.

The type of regulatory sequence, such as a promoter region, a terminator, or a secretion signal region mentioned above, is not particularly limited, and generally used promoters and secretion signal sequences can be appropriately selected depending on the host for introduction. Examples of preferred regulatory sequences that can be incorporated into the vector include the promoter, secretion signal sequence, and the like of the cellulase gene of Bacllus sp. KSM-S237 strain.

Alternatively, a marker gene (e.g., a gene resistant to drugs, such as ampicillin, neomycin, kanamycin, and chloramphenicol) for selecting the host into which the vector of the present invention is appropriately introduced may be further incorporated into the vector. Alternatively, when an auxotroph is used as the host, a gene encoding the desired nutritional synthetic enzyme may be incorporated as a marker gene into the vector. Alternatively, when a selective culture medium in which a specific metabolism is required for growth, is used, a gene associated with the metabolism may be incorporated as a marker gene into the vector. Examples of such metabolism-related gene include acetamidase genes for utilizing acetamide as a nitrogen source.

The polynucleotide encoding the mutant of the present invention, a regulatory sequence, and a marker gene can be linked by a method known in the art, such as SOE (splicing by overlap extension)-PCR (Gene, 1989, 77: 61-68). Procedures for introducing the linked fragment into the vector are well known in the art.

### <Transformed cell>

The transformed cell of the present invention can be obtained by introducing a vector containing the polynucleotide encoding the mutant of the present invention into a host, or by introducing a DNA fragment containing the polynucleotide encoding the mutant of the present invention into the genome of the host.

Examples of the host cells include microorganisms, such as bacteria and filamentous fungi. Examples of bacteria include *Escherichia coli* and bacteria belonging to the genera Staphylococcus, Enterococcus, Listeria, and Bacillus. Preferred among these are *Escherichia coli* and Bacillus bacteria (e.g., *Bacillus subtilis* Marburg No. 168 (*Bacillus subtilis* 168 strain) or mutant strains thereof). Examples of *Bacillus subtilis* mutant strains include the nine-protease-deficient strain KA8AX described in J. Biosci. Bioeng., 2007, 104(2): 135-143, and the eight-protease-deficient strain with improved protein folding efficiency, D8PA strain, described in Biotechnol. Lett., 2011, 33(9): 1847-1852. Examples of filamentous fungi include Trichoderma, Aspergillus, Rizhopus, and the like.

Methods commonly used in the art, such as the protoplast method and the electroporation method, can be used to introduce the vector into the host. Strains with appropriate introduction are selected using marker gene expression, auxotrophy, and the like as indices, whereby the target transformant into which the vector is introduced can be obtained.

Alternatively, a fragment obtained by linking the polynucleotide encoding the mutant of the present invention, a regulatory sequence, and a marker gene can also be introduced directly into the genome of the host. For example, a DNA fragment in which sequences complementary to the genome of the host are added to both ends of the linked fragment is constructed by the SOE-PCR method or the like, and this DNA fragment is then introduced into the host to induce homologous recombination between the host genome and the DNA fragment, whereby the polynucleotide encoding the mutant of the present invention is introduced into the genome of the host.

When the thus-obtained transformant into which the polynucleotide encoding the mutant of the present invention, or a vector containing the polynucleotide is introduced, is cultured in a suitable culture medium, the gene encoding the protein on the vector is expressed to produce the mutant of the present invention. The culture medium used for culturing the transformant can be appropriately selected by a person skilled in the art depending on the type of microorganism of the transformant.

Alternatively, the mutant of the present invention may be expressed from the polynucleotide encoding the mutant of the present invention or a transcript thereof using a cell-free translation system. The "cell-free translation system" is such that reagents, such as amino acids, necessary for the protein translation are added to a suspension obtained by mechanically destroying a cell, which serves as the host, to construct an in vitro transcription-translation system or an in vitro translation system.

The mutant of the present invention produced in the above culture or cell-free translation system can be isolated or purified by using general methods used for protein purification, such as centrifugation, ammonium sulfate precipitation, gel chromatography, ion-exchange chromatography, and affinity chromatography, singly or in a suitable combination. In this case, when the gene encoding the **α**-amylase mutant of the present invention is operably linked to a secretion signal sequence on the vector in the transformant, the produced protein is secreted extracellularly, and can be thus more easily collected from the culture. The protein collected from the culture may be further purified by known means.

The thus-obtained mutant of the present invention has enhanced cleaning performance and/or stability in comparison with the parent **α**-amylase.

The "enhanced cleaning performance" means an enhanced cleaning effect in comparison with the parent **α-**amylase, for example, an ability to bring about the removal of stains in the washing or cleaning step.

The cleaning performance can be evaluated by using a method well known in the art. For example, a stained cloth cut into a predetermined size is inserted into each well of a 96-well assay plate, and a cleaning agent solution and an enzyme solution are added thereto, followed by cleaning treatment under predetermined conditions. The absorbance at 488 nm of the cleaning liquid after the completion of cleaning is measured, and the difference from the blank, ΔA488, is determined as detergency. The ΔA488 of the mutant can be divided by the ΔA488 of the parent **α**-amylase to determine the relative detergency.

The "enhanced stability" means an enhanced ability to maintain **α**-amylase activity in the presence of a cleaning agent, in comparison with the parent **α**-amylase.

The stability can be evaluated by using a method well known in the art. For example, an enzyme solution is added to a cleaning agent. After treatment for a predetermined time, the **α**-amylase activity is measured, and the half-life (h) is calculated by calculating the deactivation rate per unit time (h) due to this treatment. The half-life (h) of the mutant can be divided by the half-life (h) of the parent **α**-amylase to determine the relative stability.

The mutant of the present invention is useful as an enzyme to be contained in various cleaning agent compositions, and particularly useful as an enzyme to be contained in into cleaning agent compositions suitable for low-temperature cleaning.

Examples of the "low temperature" as mentioned herein include 40°C or lower, 35°C or lower, 30°C or lower, and 25°C or lower, and also include 5°C or higher, 10°C or higher, and 15°C or higher. Other examples include from 5 to 40°C, from 10 to 35°C, from 15 to 30°C, and from 15 to 25°C.

The amount of the mutant of the present invention contained in the cleaning agent composition is not particularly limited as long as the protein can exhibit activity. For example, the amount of the mutant per kg of the cleaning agent composition is preferably 1 mg or more, more preferably 10 mg or more, and even more preferably 50 mg or more, as well as preferably 5,000 mg or less, more preferably 1,000 mg or less, and even more preferably 500 mg or less. The amount of the mutant is also preferably from 1 to 5,000 mg, more preferably from 10 to 1,000 mg, and even more preferably from 50 to 500 mg.

In the cleaning agent composition, various enzymes other than the mutant of the present invention can be used in combination. Examples thereof include hydrolases, oxidases, reductases, transferases, lyases, isomerases, ligases, synthetases, and the like. Preferred among these are amylases which are different from the protein of the present invention, proteases, cellulases, keratinases, esterases, cutinases, lipases, pullulanases, pectinases, mannanases, glucosidases, glucanases, cholesterol oxidases, peroxidases, laccases, and the like; and particularly preferred are proteases, cellulases, amylases, and lipases.

Examples of proteases include commercially available Alcalase, Esperase, Everlase, Savinase, Kannase, and Progress Uno (registered trademarks; Novozymes A/S), PREFERENZ, EFFECTENZ, and EXCELLENZ (registered trademarks; DuPont), Lavergy (registered trademark; BASF), KAP (Kao Corporation), and the like.

Examples of cellulases include Celluclean and Carezyme (registered trademarks; Novozymes A/S); KAC, the alkaline cellulase produced by Bacillus sp. KSM-S237 strain described in JP-A-10-313859, and the mutant alkaline cellulase described in JP-A-2003-313592 (Kao Corporation); and the like.

Examples of amylases include Termamyl, Duramyl, Stainzyme, Stainzyme Plus, and Amplify Prime (registered trademarks; Novozymes A/S), PREFERENZ and EFFECTENZ (registered trademarks; DuPont), KAM (Kao Corporation), and the like.

Examples of lipases include Lipolase and Lipex (registered trademarks; Novozymes A/S), and the like.

Known cleaning agent components can be contained in the cleaning agent composition, and examples of such known cleaning agent components include the following.

### (1) Surfactant

A surfactant may be contained in an amount of from 0.5 to 60 mass% in the cleaning agent composition, and preferably from 10 to 45 mass% particularly in a powder cleaning agent composition, and from 20 to 90 mass% in a liquid cleaning agent composition. When the cleaning agent composition of the present invention is a clothing cleaning agent for laundry or a cleaning agent for an automatic dishwasher, the surfactant is generally contained in an amount of from 1 to 10 mass%, and preferably from 1 to 5 mass%.

Examples of the surfactant used in the cleaning agent composition include one or a combination of anionic surfactants, nonionic surfactants, amphoteric surfactants, and cationic surfactants; and anionic surfactants and non-ionic surfactants are preferred.

Examples of preferred anionic surfactants include sulfate ester salts of alcohols having from 10 to 18 carbon atoms, sulfate ester salts of alkoxylated alcohols having from 8 to 20 carbon atoms, alkylbenzene sulfonate, paraffin sulfonate, α-olefin sulfonate, internal olefin sulfonate, α-sulfo fatty acid salts, α-sulfo fatty acid alkyl ester salts, and fatty acid salts. In the present invention, particularly preferred is one or more anionic surfactants selected from the group consisting of linear alkylbenzene sulfonate with an alkyl chain having from 10 to 14 carbon atoms, more preferably from 12 to 14 carbon atoms, and internal olefin sulfonate with an alkylene chain having from 12 to 20 carbon atoms, more preferably from 16 to 18 carbon atoms. Alkali metal salts and amines are preferable as counterions, and sodium and/or potassium, monoethanolamine, and diethanolamine are particularly preferred. For internal olefin sulfonic acid, reference can be made to, for example, WO 2017/098637.

Preferred nonionic surfactants are polyoxyalkylene alkyl (from 8 to 20 carbon atoms) ether, alkyl polyglycoside, polyoxyalkylene alkyl (from 8 to 20 carbon atoms) phenyl ether, polyoxyalkylene sorbitan fatty acid (from 8 to 22 carbon atoms) ester, polyoxyalkylene glycol fatty acid (from 8 to 22 carbon atoms) ester, and polyoxyethylene polyoxypropylene block polymers. In particular, preferred nonionic surfactants are polyoxyalkylene alkyl ethers obtained by adding 4 to 20 moles of alkylene oxides, such as ethylene oxide and propylene oxide, to alcohols having from 10 to 18 carbon atoms [an HLB value (calculated by the Griffin method) of from 10.5 to 15.0, and preferably from 11.0 to 14.5].

### (2) Divalent metal ion scavenger

A divalent metal ion scavenger may be contained in an amount of from 0.01 to 50 mass%, and preferably from 5 to 40 mass%. Examples of the divalent metal ion scavenger used in the cleaning agent composition of the present invention include condensed phosphates, such as tripolyphosphates, pyrophosphates, and orthophosphates; aluminosilicates, such as zeolites; synthetic layered crystalline silicates, nitrilotriacetates, ethylenediaminetetraacetates, citrates, isocitrates, polyacetal carboxylates, and the like. Among these, crystalline aluminosilicates (synthetic zeolites) are particularly preferred. Among A-, X-, and P-type zeolites, A-type zeolites are particularly preferred. As synthetic zeolites, those having an average primary particle size of from 0.1 to 10 µm, particularly from 0.1 to 5 µm, are preferably used.

### (3) Alkali agent

An alkali agent may be contained in an amount of from 0.01 to 80 mass%, preferably from 1 to 40 mass%. In the case of powder cleaning agents, examples of alkali agents include alkali metal carbonates, such as sodium carbonate, collectively called dense ash or light ash; and amorphous alkali metal silicates, such as JIS Nos. 1, 2, and 3. These inorganic alkali agents are effective in the formation of the particle skeleton during drying of cleaning agent, and relatively hard cleaning agents having excellent flowability can be obtained. Examples of other alkalis include sodium sesquicarbonate, sodium hydrogencarbonate, and the like. In addition, phosphates, such as tripolyphosphates, also have the action as alkali agents. As alkali agents used in liquid cleaning agents, sodium hydroxide and mono-, di-, or triethanolamine can be used, in addition to the alkali agents mentioned above, and they can also be used as counterions of activators.

### (4) Anti-redeposition agent

An anti-redeposition agent may be contained in an amount of from 0.001 to 10 mass%, preferably from 1 to 5 mass%. Examples of the anti-redeposition agent used in the cleaning agent composition of the present invention include polyethylene glycol, carboxylic acid-based polymers, polyvinyl alcohol, polyvinylpyrrolidone, and the like. Among these, carboxylic acid-based polymers have the function of scavenging metal ions and the ability to disperse solid particle stains from the clothing into the laundry bath, as well as the anti-redeposition ability. Carboxylic acid-based polymers are homopolymers or copolymers of acrylic acid, methacrylic acid, itaconic acid, or the like. Preferred copolymers are copolymers of the above monomers and maleic acid, and those with a molecular weight of from several thousands to a hundred thousand are preferred. In addition to the carboxylic acid-based polymers mentioned above, polymers such as polyglycidylates, cellulose derivatives such as carboxymethyl cellulose, and amino carboxylic acid-based polymers such as polyaspartic acid are also preferred because they have metal ion scavenging, dispersion, and anti-redeposition ability.

### (5) Bleaching agent

Ae bleaching agent, such as hydrogen peroxide or percarbonate, may be preferably contained in an amount of from 1 to 10 mass%. When the bleaching agent is used, tetraacetylethylenediamine (TAED) or the bleach activator described in JP-A-6-316700 can be contained in an amount of from 0.01 to 10 mass%.

### (6) Fluorescent agent

Examples of the fluorescent agent used in the cleaning agent composition include biphenyl-type fluorescent agents (e.g., Tinopal CBS-X and the like) and stilbene-type fluorescent agents (e.g., a DM-type fluorescent dye and the like). The fluorescent agent may be preferably contained in an amount of from 0.001 to 2 mass%.

### (7) Other components

The cleaning agent composition may contain builders, softeners, reducing agents (e.g., sulfite), foam inhibitors (e.g., silicone), fragrances, antibacterial and antifungal agents (e.g., Proxel [trade name] and benzoic acid), and other additives known in the field of clothing cleaning agents.

The cleaning agent composition can be produced in accordance with a standard method by combining the protein of the present invention obtained by the above method and the known cleaning components mentioned above. The form of the cleaning agent can be selected depending on the application. For example, the cleaning agent can be in the form of liquid, powder, granules, paste, solids, or the like.

The thus-obtained cleaning agent composition can be used as a clothing cleaning agent, a dishwashing cleaning agent, a bleaching agent, a cleaning agent for hard surface cleaning, a drain cleaning agent, a denture cleaning agent, a disinfecting cleaning agent for medical instruments, or the like; preferably a clothing cleaning agent and a dishwashing cleaning agent; and more preferably a clothing cleaning agent for laundry (laundry cleaning agent), a dishwashing cleaning agent for hand washing, and a cleaning agent for an automatic dishwasher.

The cleaning agent composition is suitable for use at 40°C or lower, 35°C or lower, 30°C or lower, or 25°C or lower, and at 5°C or higher, 10°C or higher, or 15°C or higher. The cleaning agent composition is also suitable for use at from 5 to 40°C, from 10 to 35°C, from 15 to 30°C, or from 15 to 25°C.

Regarding the embodiments described above, the present invention further discloses the following aspects.
<1> An α-amylase mutant, which is a mutant of a parent α-amylase, the α-amylase mutant comprising one or more modifications to amino acid residues at positions corresponding to positions G5, S38, T49, Q96, N126, T129, G140, F153, Q167, G179, W186, E187, N192, M199, Y200, L203, Y205, D206, R211, K215, H240, S241, Y242, G244, E257, F259, K278, H283, S284, A288, H295, Y296, N303, T320, S331, L348, Y360, W408, L429, V430, G433, A434, W439, N471, G476, and G477 in the amino acid sequence of SEQ ID NO: 2,
   the parent **α**-amylase or **α**-amylase mutant having at least 90% sequence identity to the amino acid sequence of SEQ ID NO: 4.
<2> The mutant according to <1>, wherein the modifications to amino acid residues at positions corresponding to positions G5, S38, T49, Q96, N126, T129, G140, F153, Q167, G179, W186, E187, N192, M199, Y200, L203, Y205, D206, R211, K215, H240, S241, Y242, G244, E257, F259, K278, H283, S284, A288, H295, Y296, N303, T320, S331, L348, Y360, W408, L429, V430, G433, A434, W439, N471, G476, and G477 are G5E/D/P/R/K, S38N, T49Q, Q96R/K, N126Y, T129z, G140Y/F/W, F153W, Q167E, G179D/H, W186L, E187P, N192F, M199L/T/A/N/Q/S/V/I, Y200G, L203Y/M/F, Y205F, D206R/E/N/T/G, R211V/L/I, K215F, H240F, S241A/Q/D/L/Y/P/H, Y242F, G244K/W/L/R, E257T, F259W, K278L/D/W/I/H/S/T/N/Q/V/A/Y/F, H283Q, S284W, A288F, H295Y, Y296A, N303R/E/S/G/V/D/T/A, T320D/E, S331T, L348I, Y360C/M/L/V, W408P, L429V, V430M, G433GS, A434V, W439R, N471T, G476A/P/E/S/F/R/K, and G477E, respectively.
<3> The mutant according to <1> or <2>, wherein the modifications to amino acid residues are two or more modifications.
<4> The mutant according to <1>, wherein the modifications to amino acid residues include any of modifications to amino acid residues at positions corresponding to positions N126, E187, N192, F205, R211, H240, S241, and Y242.
<5> The mutant according to <4>, wherein the modifications to amino acid residues at positions corresponding to positions N126, E187, N192, F205, R211, H240, S241, and Y242 are N126Y, E187P, N192F, F205Y, R211I, H240F, S241Q, and Y242F, respectively.
<6> The mutant according to <4> or <5>, wherein the modifications to amino acid residues are two or more modifications.
<7> The mutant according to <1> or <2>, the mutant comprising modification to one or more amino acid residues selected from the groups consisting of amino acid residues corresponding to positions E187, F205, and H240, and modification to one or more amino acid residues selected from the groups consisting of amino acid residues corresponding to positions N126, N192, R211, S241, and Y242.
<8> The mutant according to <1> or <2>, the mutant comprising modification to one or more amino acid residues selected from the groups consisting of amino acid residues corresponding to positions E187 and H240, and modification to one or more amino acid residues selected from the groups consisting of amino acid residues corresponding to positions N126, N192, F205, R211, S241, and Y242.
<9> The mutant according to <1> or <2>, the mutant comprising modification to one or more amino acid residues selected from the groups consisting of amino acid residues corresponding to positions F205 and H240, and modification to one or more amino acid residues selected from the groups consisting of amino acid residues corresponding to positions N126, E187, N192, R211, S241, and Y242.
<10> The mutant according to <1> or <2>, the mutant comprising modification to an amino acid residue corresponding to position F205, and modification to one or more amino acid residues selected from the groups consisting of amino acid residues corresponding to positions N126, E187, N192, R211, H240, S241, and Y242.
<11> The mutant according to <1> or <2>, the mutant comprising modification to an amino acid residue corresponding to position H240, and modification to one or more amino acid residues selected from the groups consisting of amino acid residues corresponding to positions N126, E187, N192, F205, R211, S241, and Y242.
<12> The mutant according to <1> or <2>, the mutant comprising modification to an amino acid residue corresponding to position R211, and modification to one or more amino acid residues selected from the groups consisting of amino acid residues corresponding to positions N126, E187, N192, F205, H240, S241, and Y242.
<13> The mutant according to <1> or <2>, wherein the parent α-amylase is an α-amylase mutant having deletion of amino acid residues at R178 and G179 in the amino acid sequence of SEQ ID NO: 2.
<14> The mutant according to <1>, <2>, or <13>, wherein the mutant comprises at least a mutation selected from the group consisting of combinations of mutations shown in the above Tables 1-1 to 1-4.
<15> The mutant according to <1>, <2>, or <13>, wherein the mutant comprises at least a mutation selected from the group consisting of combinations of mutations shown in the above Tables 2-1 to 2-4.
<16> The mutant according to <1>, <2>, or <13>, wherein the mutant comprises a mutation selected from the group consisting of combinations of mutations shown in the above Tables 1-1 to 1-4, and a mutation selected from the group consisting of combinations of mutations shown in the above Tables 2-1 to 2-4.
<17> The mutant according to <1>, <2>, or <13>, wherein the mutant comprises at least a mutation selected from the group consisting of combinations of mutations shown in the above Table 3.
<18> A polynucleotide encoding the mutant according to any one of <1> to <17>.
<19> A vector or DNA fragment comprising the polynucleotide according to <18>.
<20> A transformed cell comprising the vector or DNA fragment according to <19>.
<21> The transformed cell according to <20>, which is a microorganism.
<22> A cleaning agent composition comprising the mutant according to any one of <1> to <17>.
<23> The cleaning agent composition according to <22>, which is a clothing cleaning agent or a dishwashing cleaning agent.
<24> The cleaning agent composition according to <23>, which is a powder or a liquid.
<25> The cleaning agent composition according to any one of <22> to <24>, which is used at a low temperature.
<26> The cleaning agent composition according to <25>, which is used at a temperature of from 5 to 40°C.

### Examples

### (1) Construction of YR288 mutant expression plasmid

The method for constructing YR288 mutant described in the following examples is shown below. A forward primer having 15 bases of a sequence complementary to a reverse primer at the 5'-terminal and containing a mutant sequence, and the reverse primer having a base just before the mutant sequence at the 5'-terminal were used as a mutagenesis primer pair. The YR288 expression plasmid pHY-YR288 described in the examples of JP-A-2020-121626 or the YR288 mutant expression plasmid produced in this example was used as a template, and PCR was performed by using the mutagenesis primer pair. When multiple fragments were linked, using each of the PCR products, the In-Fusion reaction was performed in accordance with the protocol of the In-Fusion, HD Cloning kit (Clontech). With the PCR product or In-Fusion reaction solution, *Bacillus subtilis* was transformed by the protoplast method to obtain a transformant retaining the target YR288 mutant expression plasmid.

### (2) Enzyme production culture

The recombinant *Bacillus subtilis* colonies obtained in (1) were inoculated in a 96-deep-well plate into which 300 µL of LB culture medium supplemented with 15 ppm tetracycline was dispensed, and then cultured at 30°C at 210 rpm overnight. Next day, 6 µL of the culture was inoculated in a 96-deep-well plate into which 100 µL of 2xL-maltose culture medium (2% tryptone, 1% yeast extract, 1% NaCl, 7.5% maltose, 7.5 ppm manganese sulfate pentahydrate, 0.04% calcium chloride dihydrate, and 15 ppm tetracycline; % denotes (w/v)%) was dispensed, and cultured at 30°C at 210 rpm for 2 days. Then, the culture supernatant containing the enzyme produced from the bacterial cell was collected by centrifugation and used as an enzyme solution.

### (3) Measurement of protein concentration of culture supernatant

For the measurement of the protein concentration of the culture supernatant, Protein Assay Rapid Kit Wako II (FUJIFILM Wako Pure Chemical Corporation) was used. The protein concentration of a culture supernatant of a strain introduced with pHY300PLK (Takara Bio Inc.)having no amylase expression cassette was used as a blank to calculate the amylase concentration of the culture supernatant.

### (4) Activity measurement

Ethylidene-para-nitrophenyl-α-D-maltoheptaoside (Et-G7-pNP) having the non-reducing end protected was used as a substrate. Maltooligosaccharide-pNP generated by the action of α-amylase on Et-G7-pNP is subjected to the action of α-glucosidase to release pNP, and the rate of increase in the absorbance associated with pNP formation is measured to determine the α-amylase activity. A 2:1 mixture of RI and RII solutions of AMY-EL (Serotec), which is a reagent for measuring the α-amylase activity containing Et-G7-pNP and α-glucosidase, was used as a substrate solution. 100 µL of the substrate solution and 10 µL of an appropriately diluted enzyme sample were mixed in each well of a 96-well assay plate, and the absorbance change (OD/min) at 405 nm was measured at 30°C. The difference ΔOD/min from the blank (enzyme-free sample) was used as the activity value.

### (5) Stability evaluation of two-amino acid-deleted mutants at positions 178-181

The mutants shown in Fig. 1 were constructed by the method described in Example (1) using YR288 (SEQ ID NO: 2) as a parent polypeptide. An enzyme solution was added to a commercially available liquid clothing cleaning agent (Kao Corporation, Attack 3X) diluted with ion-exchange water to 10% (v/v), followed by incubation at 50°C for 15 minutes, and the activity was then measured. The remaining activity (%) was calculated by dividing the activity value of the sample after the treatment at 50°C by the activity value of the sample before the treatment at 50°C, and multiplying the resulting value by 100. The stability was significantly enhanced by deleting any two residues out of R178, G179, T180, and G181 (Fig. 1).

### (6) Detergency evaluation

A CS-26 stained cloth cut into a circular shape with a diameter of 5.5 mm was obtained from CFT. Two CS-26 circular stained cloths were inserted into each well of a 96-well assay plate, and 200 µL of a commercially available liquid clothing cleaning agent (Kao Corporation, Attack Zero) diluted 3,000-fold with tap water was added to each well. 10 µL of an enzyme solution diluted with tap water to 3 ppm was added to each well, and the plate was sealed and shaken at 20°C using a Cute Mixer at 1,200 rpm for 15 minutes. After the completion of cleaning, 100 µL of the cleaning liquid was transferred to a new 96-well assay plate, and the absorbance at 488 nm was measured. A blank was prepared by adding tap water in place of the enzyme solution, and the difference from the blank ΔA488 was determined as detergency. The ΔA488 of each mutant was divided by the ΔA488 of the parent polypeptide to determine the relative detergency.

### (7) Stability evaluation

An enzyme solution was added to a commercially available liquid clothing cleaning agent (Kao Corporation, Attack 3X or Kao Corporation, Attack Zero) diluted with ion-exchange water to 10% (v/v), followed by incubation at 50°C for from 30 minutes to 18 hours, and the activity was then measured. The activity value of the sample before the treatment at 50°C was used as the initial activity, the deactivation rate per unit time (h) by the treatment at 50°C was calculated, and the half-life (h) was calculated from the deactivation rate. The half-life (h) of each mutant was divided by the half-life (h) of the parent polypeptide to determine the relative stability.

### (8) Mutation screening

Various mutants were constructed by the method described in Example (1) using YR288 R178Δ+T180Δ (SEQ ID NO: 4) as a parent polypeptide. The performance of the mutants was evaluated by the methods described in Examples (6) and (7). When the relative detergency and/or relative stability were 1.1 or more, it was regarded that the performance was enhanced. The performance of the following mutants was enhanced.

G5E, G5D, G5P, G5R, G5K, S38N, T49Q, Q96R, Q96K, N126Y, T129I, G140Y, G140F, G140W, F153W, Q167E, G179D, G179H, W186L, E187P, N192F, M199L, M199T, M199A, M199N, M199Q, M199S, M199V, M199I, Y200G, L203Y, L203M, L203F, Y205F, D206R, D206E, D206N, D206T, D206G, R211V, R211L, R211I, K215F, H240F, S241A, S241Q, S241D, Y242F, G244K, G244W, G244L, G244R, E257T, F259W, H283Q, S284W, A288F, H295Y, Y296A, N303R, N303E, N303S, N303G, N303V, N303D, N303T, N303A, T320D, T320E, S331T, L348I, Y360C, Y360M, Y360L, Y360V, W408P, L429V, V430M, G433GS, A434V, W439R, N471T, G476A, G476P, G476E, G476S, G476F, G476R, G476K, G477E

### (9) Detergency evaluation of multiple mutants

Various mutants containing two or more of the mutations obtained in Example (8) were constructed by the method described in Example (1) using YR288 R178Δ+T180Δ (SEQ ID NO: 4) as a parent polypeptide. The detergency of the mutants was evaluated by the method described in Example (6). The results are shown below.

**[Table 4-1]**

| Enzyme (parent: R178Δ+T180Δ (SEQ ID NO: 4)) | Relative detergency |
|---|---|
| G5R+W186L+E257T | 2.7 |
| G5R+E257T+G433GS | 2.6 |
| G5R+Y360L+G433GS | 3 |
| G5R+Q96K+E257T | 2.5 |
| G5R+Y360L+W408P | 2.6 |
| G5R+F259W+S284W | 1.8 |
| G5R+W186L+F259W | 1.2 |
| G5R+S284W+T320E | 2.5 |
| Q96K+F259W+G433GS | 3 |
| Q96K+W186L+W439R | 3.2 |
| Q96K+W186L+E257T | 1.2 |
| Q96K+W408P+N471T | 2.6 |
| Q96K+E257T+W408P | 2.1 |
| Q96K+W408P+G433GS | 3 |
| Q96K+F259W+N471T | 2.8 |
| Q96K+Y360L+A434V | 2.6 |
| Q96K+F259W+W408P | 2.9 |
| W186L+E257T+Y360L | 2.8 |
| W186L+W408P+N471T | 1.7 |
| W186L+E257T+W408P | 1.3 |
| W186L+E257T+N471T | 2.5 |
| E257T+A434V+N471T | 1.1 |
| E257T+W408P+A434V | 2.1 |
| F259W+S284W+W439R | 1.7 |
| F259W+S284W+Y360L | 2.2 |
| F259W+T320E+G433GS | 2.3 |
| S284W+T320E+Y360L | 1.9 |
| S284W+W408P+G433GS | 2.6 |
| S284W+T320E+A434V | 2.2 |
| T320E+Y360L+G433GS | 1.8 |

**[Table 4-2]**

| Enzyme (parent: R178Δ+T180Δ (SEQ ID NO: 4)) | Relative detergency |
|---|---|
| T320E+W439R+N471T | 1.9 |
| T320E+G433GS+N471T | 2.1 |
| T320E+Y360L+W439R | 2.6 |
| Y360L+A434V+N471T | 2.2 |
| G5R+S284W+W439R | 3.1 |
| Q96K+E257T+T320E+W408P | 2.6 |
| Q96K+E257T+W408P+A434V | 2.1 |
| Q96K+E257T+W408P+N471T | 2.5 |
| Q96K+E257T+T320E+W408P+A434V | 2.9 |
| Q96K+E257T+T320E+W408P+N471T | 2.9 |
| Q96K+T320E+Y360L+W408P+A434V | 2.3 |
| Q96K+T320E+Y360L+A434V+N471T | 2.1 |
| E257T+T320E+W408P+A434V+N471T | 2.8 |
| G5R+Q96K+W186L+E257T+T320E+W439R | 2.3 |
| G5R+Q96K+W186L+E257T+S284W+G433GS | 1.4 |
| G5R+F259W+S284W+T320E+W439R+N471T | 2.5 |
| G5R+Q96K+W186L+Y360L+W408P+G433GS | 3.3 |
| G5R+Q96K+W186L+E257T+W408P+W439R | 1.9 |
| G5R+Q96K+F259W+Y360L+W408P+W439R | 3 |
| G5R+E257T+F259W+S284W+Y360L+N471T | 2.8 |
| G5R+Q96K+W186L+F259W+Y360L+G433GS | 3.2 |
| G5R+F259W+S284W+T320E+Y360L+A434V | 2.2 |
| Q96K+E257T+F259W+T320E+G433GS+N471T | 2.8 |
| Q96K+W186L+E257T+S284W+W439R+N471T | 3.1 |
| Q96K+S284W+T320E+W408P+W439R+N471T | 3 |
| Q96K+W186L+E257T+W408P+A434V+N471T | 3.3 |
| Q96K+W186L+F259W+Y360L+W439R+N471T | 3.4 |
| G5R+Q96K+Y360L+W408P+G433GS | 3.1 |
| G5R+Q96K+F259W+Y360L+G433GS | 3.1 |
| Q96K+F259W+Y360L+W439R+N471T | 3 |

**[Table 4-3]**

| Enzyme (parent: R178Δ+T180Δ (SEQ ID NO: 4)) | Relative detergency |
|---|---|
| Q96K+E257T+W408P+A434V+N471T | 2.6 |
| E257T+Y360L | 2.6 |
| E257T+Y360L+W408P | 2.8 |
| E257T+Y360L+G476K | 3.1 |
| E257T+Y360L+W408P+G476K | 3.3 |
| G5R+S38N | 2.2 |
| Q96K+S38N | 2.1 |
| S38N+E257T | 2.2 |
| S38N+F259W | 2.2 |
| S38N+S284W | 2.6 |
| S38N+T320D | 2.4 |
| S38N+T320E | 2 |
| S38N+Y360L | 2.3 |
| S38N+W408P | 2 |
| S38N+N471T | 2 |
| S38N+G476A | 1.9 |
| S38N+G476K | 2.2 |
| S38N+G476E | 1.9 |
| S38N+N471T+G476K | 2.5 |
| S38N+N471T+G476K+G477E | 2.6 |
| E257T+Y360C | 1.9 |
| E257T+Y360M | 2.5 |
| E257T+Y360V | 2.3 |
| E257T+G476K+Y360C | 2.5 |
| E257T+G476K+Y360M | 3.1 |
| E257T+G476K+Y360V | 2.7 |
| G5R+W186L | 3.1 |
| G5R+E257T | 1.8 |
| G5R+Y360L | 2 |
| S284W+T320E | 2 |
| Q96K+F259W | 2.2 |

**[Table 4-4]**

| Enzyme (parent: R178Δ+T180Δ (SEQ ID NO: 4)) | Relative detergency |
|---|---|
| Q96K+W186L | 2 |
| Q96K+W408P | 2.1 |
| G5R+S284W | 2.4 |
| G5R+W439R | 3 |
| S38N+S284W | 2.6 |
| S38N+T320D | 2.4 |

### (10) Stability evaluation of multiple mutants

Various mutants containing two or more of the mutations obtained in Example (8) were constructed by the method described in Example (1) using YR288 R178Δ+T180Δ (SEQ ID NO: 4) as a parent polypeptide. The stability of the mutants was evaluated by the method described in Example (7). The results are shown below.

**[Table 5-1]**

| Enzyme (parent: R178Δ+T180Δ (SEQ ID NO: 4)) | Relative stability |
|---|---|
| F205Y+H240F+Y242F | 17.1 |
| N192F+F205Y+H240F+Y242F | 34.6 |
| E187P+N192F | 66.3 |
| H240F+Y242F | 9.7 |
| H240F+Y242F+S331T | 15.7 |
| N126Y+T129I+L203Y+F205Y | 4.3 |
| N126Y+T1291+H240F+Y242F+G244W | 27.2 |
| N126Y+T129I+H283Q+A288F | 7 |
| H240F+Y242F+G244W | 9.6 |
| H240F+G244W | 7.8 |
| Y242F+G244W | 1.8 |
| L203Y+F205Y | 1.7 |
| N126Y+T129I | 2.5 |
| H283Q+A288F | 2.8 |
| S241Q+Y242F | 7.5 |
| S241F+G244W | 8.2 |
| S241Q+Y242F+G244W | 7.7 |
| H240F+S241Q+G244W | 40 |
| H240F+S241Q+Y242F | 39.7 |
| T129I+E187P+G244W+S331T | 40.7 |
| S241Q+H283Q+A288F+S331T | 10.4 |
| N126Y+T1291+G140W+E187P | 61.6 |
| N126Y+G179D+N192F+L203Y | 3.1 |
| N126Y+F205Y+Y242F+A288F | 5.7 |
| L203Y+R211I+Y242F+H283Q | 3.3 |
| G179D+E187P+R211I+H240F | 44.6 |
| G140W+L203Y+Y242F+G244W | 2.7 |
| H240F+S241Q+Y242F+G244W | 38.7 |
| F205Y+H240F+S241Q | 47.3 |
| F205Y+H240F+S241Q+Y242F | 57.9 |

**[Table 5-2]**

| Enzyme (parent: R178Δ+T180Δ (SEQ ID NO: 4)) | Relative stability |
|---|---|
| F205Y+S241Q | 7.8 |
| E187P+H240F+S241Q | 38.9 |
| E187P+H240F+S241Q+Y242F | 43 |
| E187P+S241Q+Y242F | 18 |
| E187P+H240F+Y242F | 73.5 |
| N126Y+E187P | 62.1 |
| T129I+E187P | 36.7 |
| G140W+E187P | 36.7 |
| E187P+L203Y | 42.6 |
| E187P+F205Y | 35.3 |
| E187P+S241Q | 34.6 |
| E187P+Y242F | 36.9 |
| E187P+G244W | 36.6 |
| E187P+H283Q | 34.5 |
| E187P+S331T | 44.6 |
| N126Y+T129I+E187P | 64.7 |
| E187P+N192F+F205Y+H240F+Y242F | 99.2 |
| M199L+F205Y+R211I+H240F+S241Q+Y242F | 112.9 |
| H240F+S241Q | 33 |
| E187P+R211I | 61.1 |
| E187P+N192F+M199L | 88.1 |
| E187P+F205Y+H240F+Y242F | 81.7 |
| E187P+N192F+Y242F | 61.1 |
| E187P+N192F+R211I | 119.6 |
| E187P+N192F+M199L+R211I | 88.4 |
| E187P+N192F+F205Y+Y242F | 70.6 |
| E187P+N192F+F205Y+H240F+Y242F | 77.9 |

**[Table 5-3]**

| Enzyme (parent: R178Δ+T180Δ (SEQ ID NO: 4)) | Relative stability |
|---|---|
| N192F+S241Q | 7.9 |
| N192F+H240F+S241Q | 60.7 |
| N192F+F205Y+H240F+S241Q | 71.3 |
| N192F+F205Y+H240F+S241Q+Y242F | 69.9 |
| R211I+H240F+S241Q | 76.3 |
| R211I+H240F+S241Q+Y242F | 77.2 |
| F205Y+R211I+H240F+S241Q | 83 |
| F205Y+R211I+H240F+S241Q+Y242F | 94.8 |
| G179H+M199L+F205Y+R211I+H240F+S241Q+Y242F | 99.8 |
| G179H+F205Y+R211I+H240F+S241Q+Y242F | 76.2 |
| G179H+F205Y+H240F+S241Q+Y242F | 56.7 |
| G179H+E187P+N192F | 65.7 |
| E187P+M199L | 45.3 |
| M199L+H240F+S241Q | 40.6 |
| F205Y+H240F+S241A | 17.1 |
| F205Y+H240F+S241D | 87.4 |
| H240F+S241A | 15.1 |
| H240F+S241D | 77 |
| N126Y+R211I | 5.2 |
| N126Y+H240F | 13.1 |
| E187P+H240F | 76.9 |
| N192F+F205Y | 3.6 |
| N192F+R211I | 9.2 |
| N192F+H240F | 20 |
| F205Y+R211I | 6.4 |
| F205Y+H240F | 12.1 |
| R211I+H240F | 23 |
| R211I+S241Q | 18 |
| R211I+Y242F | 5.8 |

**[Table 5-4]**

| Enzyme (parent: R178Δ+T180Δ (SEQ ID NO: 4)) | Relative stability |
|---|---|
| N192F+M199L+F205Y+R211I+H240F+S241Q+Y242F | 84.8 |
| G179H+N192F+M199L+F205Y+R211I+H240F+S241Q+Y242F | 147.8 |
| N192F+F205Y+R211I+H240F+S241Q+Y242F | 116.7 |
| G179H+N192F+F205Y+R211I+H240F+S241Q+Y242F | 98.7 |
| N192F+R211I+H240F+S241Q+Y242F | 107.8 |
| N192F+F205Y+R211I+H240F+S241Q | 102.6 |

Various mutants were constructed by the method described in Example (1) using YR288 R178Δ+T180Δ (SEQ ID NO: 4) as a parent polypeptide. The stability of the mutants was evaluated by the method described in Example (7). The results are shown below.

**[Table 5-5]**

| Enzyme (parent: R178Δ+T180Δ (SEQ ID NO: 4)) | Relative stability |
|---|---|
| E187P+K278L | 81.8 |
| E187P+K278D | 56.5 |
| E187P+K278W | 106 |
| E187P+K278I | 82.2 |
| E187P+K278H | 80.3 |
| E187P+K278S | 54.1 |
| E187P+K278T | 63.4 |
| E187P+K278N | 47.5 |
| E187P+K278Q | 62.1 |
| E187P+K278V | 63 |
| E187P+K278A | 50.1 |
| E187P+K278Y | 114.9 |
| E187P+K278F | 102.4 |
| E187P+S241L+K278W | 131.2 |
| E187P+S241Y+K278I | 75.1 |
| E187P+S241P+K278W | 40.7 |
| E187P+S241L+K278I | 37.9 |
| E187P+S241P+K278I | 102.6 |
| E187P+S241Y+K278W | 47.6 |
| E187P+S241F+K278W | 78.6 |
| E187P+S241Y+K278Y | 109.7 |
| E187P+S241Y+K278F | 105.8 |
| E187P+S241Y+K278H | 47.9 |
| E187P+S241Y+K278L | 49.8 |
| E187P+S241H+K278W | 96.6 |

### (11) Evaluation of detergency and stability of multiple mutants

Various mutants containing the multiple mutation obtained in Example (9) and the multiple mutation obtained in Example (10) were constructed by the method described in Example (1) using YR288 R178Δ+T180Δ (SEQ ID NO: 4) as a parent polypeptide. The performance of the mutants was evaluated by the methods described in Examples (6) and (7). The results are shown below.

**[Table 6]**

| Enzyme (parent: R178Δ+T180Δ (SEQ ID NO: 4)) | Relative detergency | Relative stability |
|---|---|---|
| G5R+E187P+N192F+S284W+W439R | 2.9 | 58.3 |
| G5R+E187P+N192F+Y360L+G433GS | 2.8 | 54.3 |
| Q96K+E187P+N192F+F259W+G433GS | 2.5 | 69.7 |
| F205Y+H240F+S241Q+Y242F+E257T+Y360L | 2.4 | 47.6 |
| F205Y+H240F+S241Q+Y242F+E257T+Y360L+W408P | 2.6 | 49.1 |
| F205Y+H240F+S241Q+Y242F+E257T+Y360L+G476K | 3.1 | 48.1 |
| F205Y+R211I+H240F+S241Q+Y242F+E257T+Y360L | 2.3 | 74.9 |
| F205Y+H240F+S241Q+Y242F+E257T+Y360L+W408P+G476K | 3 | 45.8 |
| M199L+F205Y+H240F+S241Q+Y242F+E257T+Y360L+W408P+G476K | 2.9 | 57.3 |
| M199L+F205Y+H240F+Y242F+E257T+Y360L+S241Q+G476K | 2.8 | 54.7 |
| M199L+F205Y+R211I+H240F+S241Q+Y242F+E257T+Y360L+W408P+G476K | 2.8 | 104.6 |
| M199L+F205Y+H240F+S241Q+Y242F+E257T+S331T+Y360L+W408P+G476K | 2.7 | 66.5 |
| N126Y+M199L+F205Y+H240F+S241Q+Y242F+E257T+Y360L+W408P+G476K | 2.8 | 79.8 |
| G179H+N192F+M199L+F205Y+R211I+H240F+S241Q+Y242F+E257T+Y360L +W408P+G476K | 3 | 115.2 |
| S38N+G179H+N192F+M199L+F205Y+R211I+H240F+S241Q+Y242F+N471T +G476K+G477E | 2.1 | 113.3 |
| Q96K+E187P+N192F+R211I+F259W+G433GS | 3.3 | 169.4 |
| S38N+M199L+F205Y+R211I+H240F+S241Q+Y242F+N471T+G476K+G477E | 2.2 | 100.4 |
| Q96K+G179H+N192F+M199L+F205Y+R2111+H240F+S241Q+Y242F+F259W +G433GS | 2.5 | 274.4 |
| E187P+N192F+R211I+E257T+Y360L+W408P+G476K | 3.5 | 107.8 |
| Q96K+M199L+F205Y+R211I+H240F+S241Q+Y242F+F259W+G433GS | 2.8 | 182.7 |
| G5R+M199L+F205Y+R211I+H240F+S241Q+Y242F+S284W+W439R | 2.8 | 104.4 |
| G5R+G179H+N192F+M199L+F205Y+R2111+H240F+S241Q+Y242F+S284W +W439R | 2.4 | 146 |

### (12) Evaluation of detergency and stability of multiple mutants

The following mutants were constructed by the method described in Example (1) using YR288 R178Δ+G179Δ as a parent polypeptide. The performance of the mutants was evaluated by the methods described in Examples (6) and (7). The results are shown below.

**[Table 7]**

| Enzyme (parent: R178Δ+G179Δ) | Relative detergency | Relative stability |
|---|---|---|
| G5R+E 187P+N 192 F+S284W+W439R | 2.7 | 59.4 |
| G5R+E187P+N192F+Y360L+G433GS | 2.7 | 58.4 |
| Q96K+E187P+N192F+F259W+G433GS | 2.5 | 75.2 |
| G5R+E187P+N192F+M199L+S284W+W439R | 2.2 | 67.9 |
| E187P+M199L+F205Y+H240F+S241Q+Y242F+E257T+Y360L+G476K+W408P | 3.2 | 57.4 |
| G5R+Q96K+E187P+N192F+M199L+Y242F+F259W+S331T+Y360L+W439R | 2 | 83.5 |
| G5R+Q96K+E187P+N192F+M199L+R211I+Y242F+F259W+Y360L+W439R | 2.9 | 200.4 |
| M199L+F205Y+R211I+H240F+S241Q+Y242F+E257T+Y360L+W408P+G476K | 3.1 | 116 |

## Claims

1. An α-amylase mutant, which is a mutant of a parent α-amylase, the α-amylase mutant comprising one or more modifications to amino acid residues at positions corresponding to positions G5, S38, T49, Q96, N126, T129, G140, F153, Q167, G179, W186, E187, N192, M199, Y200, L203, Y205, D206, R211, K215, H240, S241, Y242, G244, E257, F259, K278, H283, S284, A288, H295, Y296, N303, T320, S331, L348, Y360, W408, L429, V430, G433, A434, W439, N471, G476, and G477 in the amino acid sequence of SEQ ID NO: 2,
the parent α-amylase or α-amylase mutant having at least 90% sequence identity to the amino acid sequence of SEQ ID NO: 4.

2. The mutant according to claim 1, wherein the modifications to amino acid residues at positions corresponding to positions G5, S38, T49, Q96, N126, T129, G140, F153, Q167, G179, W186, E187, N192, M199, Y200, L203, Y205, D206, R211, K215, H240, S241, Y242, G244, E257, F259, K278, H283, S284, A288, H295, Y296, N303, T320, S331, L348, Y360, W408, L429, V430, G433, A434, W439, N471, G476, and G477 are G5E/D/P/R/K, S38N, T49Q, Q96R/K, N126Y, T129I, G140Y/F/W, F153W, Q167E, G179D/H, W186L, E187P, N192F, M199L/T/A/N/Q/S/V/I, Y200G, L203Y/M/F, Y205F, D206R/E/N/T/G, R211V/L/I, K215F, H240F, S241A/Q/D/L/Y/P/H, Y242F, G244K/W/L/R, E257T, F259W, K278L/D/W/I/H/S/T/N/Q/V/A/Y/F, H283Q, S284W, A288F, H295Y, Y296A, N303R/E/S/G/V/D/T/A, T320D/E, S331T, L348I, Y360C/M/L/V, W408P, L429V, V430M, G433GS, A434V, W439R, N471T, G476A/P/E/S/F/R/K, and G477E, respectively.

3. The mutant according to claim 1 or 2, wherein the modifications to amino acid residues are two or more modifications.

4. The mutant according to claim 1 or 2, wherein the mutant comprises at least a mutation selected from the group consisting of combinations of mutations shown in the following Tables 1-1 to 1-4.
| [Table 1-1] | [Table 1-2] |
|---|---|
| G5R+W186L+E257T | T320E+W439R+N471T |
| G5R+E257T+G433GS | T320E+G433GS+N471T |
| G5R+Y360L+G433GS | T320E+Y360L+W439R |
| G5R+Q96K+E257T | Y360L+A434V+N471T |
| G5R+Y360L+W408P | G5R+S284W+W439R |
| G5R+F259W+S284W | Q96K+E257T+T320E+W408P |
| G5R+W186L+F259W | Q96K+E257T+W408P+A434V |
| G5R+S284W+T320E | Q96K+E257T+W408P+N471T |
| Q96K+F259W+G433GS | Q96K+E257T+T320E+W408P+A434V |
| Q96K+W186L+W439R | Q96K+E257T+T320E+W408P+N471T |
| Q96K+W186L+E257T | Q96K+T320E+Y360L+W408P+A434V |
| Q96K+W408P+N471T | Q96K+T320E+Y360L+A434V+N471T |
| Q96K+E257T+W408P | E257T+T320E+W408P+A434V+N471T |
| Q96K+W408P+G433GS | G5R+Q96K+W186L+E257T+T320E+W439R |
| Q96K+F259W+N471T | G5R+Q96K+W186L+E257T+S284W+G433GS |
| Q96K+Y360L+A434V | G5R+F259W+S284W+T320E+W439R+N471T |
| Q96K+F259W+W408P | G5R+Q96K+W186L+Y360L+W408P+G433GS |
| W186L+E257T+Y360L | G5R+Q96K+W186L+E257T+W408P+W439R |
| W186L+W408P+N471T | G5R+Q96K+F259W+Y360L+W408P+W439R |
| W186L+E257T+W408P | G5R+E257T+F259W+S284W+Y360L+N471T |
| W186L+E257T+N471T | G5R+Q96K+W186L+F259W+Y360L+G433GS |
| E257T+A434V+N471T | G5R+F259W+S284W+T320E+Y360L+A434V |
| E257T+W408P+A434V | Q96K+E257T+F259W+T320E+G433GS+N471T |
| F259W+S284W+W439R | Q96K+W186L+E257T+S284W+W439R+N471T |
| F259W+S284W+Y360L | Q96K+S284W+T320E+W408P+W439R+N471T |
| F259W+T320E+G433GS | Q96K+W186L+E257T+W408P+A434V+N471T |
| S284W+T320E+Y360L | Q96K+W186L+F259W+Y360L+W439R+N471T |
| S284W+W408P+G433GS | G5R+Q96K+Y360L+W408P+G433GS |
| S284W+T320E+A434V | G5R+Q96K+F259W+Y360L+G433GS |
| T320E+Y360L+G433GS | Q96K+F259W+Y360L+W439R+N471T |
| [Table 1-3] | [Table 1-4] |
|---|---|
| Q96K+E257T+W408P+A434V+N471T | Q96K+W186L |
| E257T+Y360L | Q96K+W408P |
| E257T+Y360L+W408P | G5R+S284W |
| E257T+Y360L+G476K | G5R+W439R |
| E257T+Y360L+W408P+G476K | S38N+S284W |
| G5R+S38N | S38N+T320D |
| Q96K+S38N | |
| S38N+E257T | |
| S38N+F259W | |
| S38N+S284W | |
| S38N+T320D | |
| S38N+T320E | |
| S38N+Y360L | |
| S38N+W408P | |
| S38N+N471T | |
| S38N+G476A | |
| S38N+G476K | |
| S38N+G476E | |
| S38N+N471T+G476K | |
| S38N+N471T+G476K+G477E | |
| E257T+Y360C | |
| E257T+Y360M | |
| E257T+Y360V | |
| E257T+G476K+Y360C | |
| E257T+G476K+Y360M | |
| E257T+G476K+Y360V | |
| G5R+W186L | |
| G5R+E257T | |
| G5R+Y360L | |
| S284W+T320E | |
| Q96K+F259W | |

5. The mutant according to claim 1 or 2, wherein the mutant comprises at least a mutation selected from the group consisting of combinations of mutations shown in the following Tables 2-1 to 2-4.
| [Table 2-1] | [Table 2-2] |
|---|---|
| F205Y+H240F+Y242F | F205Y+S241Q |
| N192F+F205Y+H240F+Y242F | E187P+H240F+S241Q |
| E187P+N192F | E187P+H240F+S241Q+Y242F |
| H240F+Y242F | E187P+S241Q+Y242F |
| H240F+Y242F+S331T | E187P+H240F+Y242F |
| N126Y+T129I+L203Y+F205Y | N126Y+E187P |
| N126Y+T1291+H240F+Y242F+G244W | T129I+E187P |
| N126Y+T129I+H283Q+A288F | G140W+E187P |
| H240F+Y242F+G244W | E187P+L203Y |
| H240F+G244W | E187P+F205Y |
| Y242F+G244W | E187P+S241Q |
| L203Y+F205Y | E187P+Y242F |
| N126Y+T129I | E187P+G244W |
| H283Q+A288F | E187P+H283Q |
| S241Q+Y242F | E187P+S331T |
| S241 F+G244W | N126Y+T129I+E187P |
| S241Q+Y242F+G244W | E187P+N192F+F205Y+H240F+Y242F |
| H240F+S241Q+G244W | M1 99L+F205Y+R21 1 I+H240F+S241 Q+Y242F |
| H240F+S241Q+Y242F | H240F+S241Q |
| T129I+E187P+G244W+S331T | E187P+R211I |
| S241Q+H283Q+A288F+S331T | E187P+N192F+M199L |
| N126Y+T129I+G140W+E187P | E187P+F205Y+H240F+Y242F |
| N126Y+G179D+N192F+L203Y | E187P+N192F+Y242F |
| N126Y+F205Y+Y242F+A288F | E187P+N192F+R211I |
| L203Y+R211I+Y242F+H283Q | E187P+N192F+M199L+R211I |
| G179D+E187P+R211I+H240F | E187P+N192F+F205Y+Y242F |
| G140W+L203Y+Y242F+G244W | E187P+N192F+F205Y+H240F+Y242F |
| H240F+S241Q+Y242F+G244W | |
| F205Y+H240F+S241Q | |
| F205Y+H240F+S241Q+Y242F | |
| [Table 2-3] | [Table 2-4] |
|---|---|
| N192F+S241Q | N192F+M199L+F205Y+R211I+H240F+S241Q+Y242F |
| N192F+H240F+S241Q | G179H+N192F+M199L+F205Y+R211I+H240F+S241Q +Y242F |
| N192F+F205Y+H240F+S241Q | N192F+F205Y+R211I+H240F+S241Q+Y242F |
| N192F+F205Y+H240F+S241Q+Y242F | G179H+N192F+F205Y+R211I+H240F+S241Q+Y242F |
| R211I+H240F+S241Q | N192F+R211I+H240F+S241Q+Y242F |
| R2111+H240F+S241 Q+Y242F | N192F+F205Y+R211I+H240F+S241Q |
| F205Y+R2111+H240F+S241Q | E187P+K278L |
| F205Y+R211I+H240F+S241Q+Y242F | E187P+K278D |
| G179H+M199L+F205Y+R211I+H240F+S241Q+Y242F | E187P+K278W |
| G179H+F205Y+R211I+H240F+S241Q+Y242F | E187P+K278I |
| G179H+F205Y+H240F+S241Q+Y242F | E187P+K278H |
| G179H+E187P+N192F | E187P+K278S |
| E187P+M199L | E187P+K278T |
| M199L+H240F+S241Q | E187P+K278N |
| F205Y+H240F+S241A | E187P+K278Q |
| F205Y+H240F+S241D | E187P+K278V |
| H240F+S241A | E187P+K278A |
| H240F+S241D | E187P+K278Y |
| N126Y+R211I | E187P+K278F |
| N126Y+H240F | E187P+S241L+K278W |
| E187P+H240F | E187P+S241Y+K278I |
| N192F+F205Y | E187P+S241P+K278W |
| N192F+R211I | E187P+S241L+K278I |
| N192F+H240F | E187P+S241P+K278I |
| F205Y+R211I | E187P+S241Y+K278W |
| F205Y+H240F | E187P+S241F+K278W |
| R211I+H240F | E187P+S241Y+K278Y |
| R211I+S241Q | E187P+S241Y+K278F |
| R211I+Y242F | E187P+S241Y+K278H |
| | E187P+S241Y+K278L |
| | E187P+S241H+K278W |

6. The mutant according to claim 1 or 2, wherein the mutant comprises at least a mutation selected from the group consisting of combinations of mutations shown in the following Table 3.
| [Table 3] |
|---|
| G5R+E187P+N 192F+S284W+W439R |
| G5R+E187P+N192F+Y360L+G433GS |
| Q96K+E187P+N192F+F259W+G433GS |
| F205Y+H240F+S241Q+Y242F+E257T+Y360L |
| F205Y+H240F+S241Q+Y242F+E257T+Y360L+W408P |
| F205Y+H240F+S241Q+Y242F+E257T+Y360L+G476K |
| F205Y+R211I+H240F+S241Q+Y242F+E257T+Y360L |
| F205Y+H240F+S241Q+Y242F+E257T+Y360L+W408P+G476K |
| M199L+F205Y+H240F+S241Q+Y242F+E257T+Y360L+W408P+G476K |
| M199L+F205Y+H240F+Y242F+E257T+Y360L+S241Q+G476K |
| M199L+F205Y+R211I+H240F+S241Q+Y242F+E257T+Y360L+W408P+G476K |
| M199L+F205Y+H240F+S241Q+Y242F+E257T+S331T+Y360L+W408P+G476K |
| N126Y+M199L+F205Y+H240F+S241Q+Y242F+E257T+Y360L+W408P+G476K |
| E187P+M199L+F205Y+H240F+S241Q+Y242F+E257T+Y360L+G476K+W408P |
| G5R+Q96K+E187P+N192F+M199L+Y242F+F259W+S331T+Y360L+W439R |
| G5R+Q96K+E187P+N192F+M199L+R211I+Y242F+F259W+Y360L+W439R |
| G179H+N192F+M199L+F205Y+R211I+H240F+S241Q+Y242F+E257T+Y360L+W408P+G476K |
| S38N+G179H+N192F+M199L+F205Y+R211I+H240F+S241Q+Y242F+N471T+G476K+G477E |
| Q96K+E187P+N192F+R211I+F259W+G433GS |
| S38N+M199L+F205Y+R211I+H240F+S241Q+Y242F+N471T+G476K+G477E |
| Q96K+G179H+N192F+M199L+F205Y+R211I+H240F+S241Q+Y242F+F259W+G433GS |
| E187P+N192F+R211I+E257T+Y360L+W408P+G476K |
| Q96K+M199L+F205Y+R211I+H240F+S241Q+Y242F+F259W+G433GS |
| G5R+M199L+F205Y+R211I+H240F+S241Q+Y242F+S284W+W439R |
| G5R+G179H+N192F+M199L+F205Y+R211I+H240F+S241Q+Y242F+S284W+W439R |

7. A polynucleotide encoding the mutant according to any one of claims 1 to 6.

8. A vector or DNA fragment comprising the polynucleotide according to claim 7.

9. A transformed cell comprising the vector or DNA fragment according to claim 8.

10. The transformed cell according to claim 9, which is a microorganism.

11. A cleaning agent composition comprising the mutant according to any one of claims 1 to 6.

12. The cleaning agent composition according to claim 11, which is a clothing cleaning agent or a dishwashing cleaning agent.

13. The cleaning agent composition according to claim 12, which is a powder or a liquid.

14. The cleaning agent composition according to claim 12 or 13, which is used at a low temperature.

15. The cleaning agent composition according to claim 14, which is used at a temperature of from 5 to 40°C.
